# EUROPEAN PATENT APPLICATION

(11) **EP 4 800 000 A1**
(43) Date of publication of application: **02.09.2026**
(21) Application number: 25160829.5
(22) Date of filing: 28.02.2025
(51) Int. Cl.: C07C 29/151, C07C 31/04, C25B 1/04, C25B 1/23

(54) **PLANT AND METHOD FOR CONVERTING CO2 TO METHANOL**

(71) Applicant: Topsoe A/S, 2800 Kgs. Lyngby (DK)
(72) Inventor: PASSEY, Pavni, 121003 Haryana (IN); FRIIS-CHRISTENSEN, Troel, Juel, 2800 Kgs. Lyngby (DK); SØRENSEN, Per, Aggerholm, 2800 Kgs. Lyngby (DK)
(74) Representative: Topsoe A/S

(57) **Abstract**

The invention relates to an methanol plant comprising: a CO₂-rich feed, a hydrogen-rich feed, a boiler feed water stream, a CO₂-electrolysis section arranged to electrolyse at least a portion of the CO₂-rich feed to output a mixed stream, a heat exchange section arranged to heat exchange at least a portion of the first mixed stream so as to output a cooled mixed stream and a steam stream, a methanol synthesis loop arranged to receive at least a portion of the cooled mixed stream and at least a portion of the hydrogen-rich feed and to output a raw methanol stream, a purge gas stream, and a flash gas stream, and a methanol upgrading section, wherein the steam stream is arranged to provide heat energy for one or more components of the methanol upgrading section. At least a portion of the purge gas stream is arranged to be recycled to the CO₂-rich feed, and/or at least a portion of the flash gas stream is arranged to be recycled to at least one of: the mixed stream and the cooled mixed stream. The invention also relates to a method for converting CO₂ to methanol.

## Description

### FIELD OF THE INVENTION

The present invention relates to a methanol plant and a method for converting CO₂ to methanol.

### BACKGROUND

Carbon capture, carbon utilisation, and carbon storage technologies are effective to mitigate the greenhouse effect. Captured CO₂ can be utilised by converting it to methanol. A standard synthesis route for methanol production from CO₂ is by direct hydrogenation of CO₂. This process typically requires a high import of steam to close the heat balance of the process.

There is a need for a plant and method for converting CO₂ to methanol with reduced import of steam.

It is a further object to provide a plant and method for converting CO₂ to methanol using renewable energy. It is a further object to provide a plant and method for converting CO₂ to methanol with reduced feed requirements and a reduced methanol synthesis loop size, compared to methanol production by direct hydrogenation of CO₂. Finally, it is an object to provide an energy-efficient plant and method for converting CO₂ to methanol.

### SUMMARY

Embodiments of the invention generally relate to a methanol plant and method for converting CO₂ to methanol.

In a first aspect, the invention relates to a methanol plant, the methanol plant comprising:
- a CO₂-rich feed,
- a hydrogen-rich feed,
- a boiler feed water stream,
- a CO₂-electrolysis section arranged to receive the CO₂-rich feed, to electrolyse at least a portion of the CO₂-rich feed, and to output a mixed stream comprising CO and CO₂,
- a heat exchange section arranged to heat exchange at least a portion of the mixed stream with the boiler feed water stream so as to output a cooled mixed stream and a steam stream from the heat exchange section,
- a methanol synthesis loop arranged to receive at least a portion of the cooled mixed stream and the hydrogen-rich feed - preferably in admixture - and to output a raw methanol stream, a purge gas stream, and a flash gas stream,
- a methanol upgrading section arranged to receive at least a portion of the raw methanol stream and to output an upgraded methanol stream and an off-gas stream,
wherein at least a portion of the steam stream is arranged to provide heat energy for one or more components of the methanol upgrading section, and wherein
at least a portion of the purge gas stream is arranged to be recycled to the mixed stream and/or the cooled mixed stream,
   and/or
at least a portion of the flash gas stream is arranged to be recycled to the mixed stream and/or the cooled mixed stream.

Also provided is a method for converting CO₂ to methanol, comprising the steps of:
a) providing a CO₂-rich feed,
b) providing a hydrogen-rich feed,
c) providing a boiler feed water stream,
d) supplying the CO₂-rich feed to a CO₂-electrolysis section,
e) electrolysing at least a portion of the CO₂-rich feed in the CO₂-electrolysis section to output a mixed stream comprising CO and CO₂ from the CO₂-electrolysis section from the CO₂-electrolysis section,
f) heat exchanging at least a portion of the mixed stream with the boiler feed water stream in a heat exchange section to output a cooled mixed stream and a steam stream from the heat exchange section,
g) supplying at least a portion of the cooled mixed stream and the hydrogen-rich feed - preferably in admixture - to a methanol synthesis loop to output a raw methanol stream, a purge gas stream, and a flash gas stream, from the methanol synthesis loop,
h) supplying at least a portion of the raw methanol stream to a methanol upgrading section to output an upgraded methanol stream and an off-gas stream from the methanol upgrading section, and
i) using at least a portion of the steam stream to provide heat energy for one or more components of the methanol upgrading section, and
j) one or more of the following steps:
   - recycling at least a portion of the purge gas stream to the mixed stream and/or the cooled mixed stream,
      and/or
   - recycling at least a portion of the flash gas stream to the mixed stream and/or the cooled mixed stream.

Further details of the invention are provided in the following description and figures.

### BRIEF DESCRIPTION OF THE DRAWINGS

Fig. 1 shows a first embodiment of the plant and method disclosed herein.
Fig. 2 shows a further embodiment of the plant and method disclosed herein.

### DETAILED DISCLOSURE

The term 'flash gas' denotes a gas obtained by degassing raw methanol through a reduction in pressure of the raw methanol. The gas contains primarily CO₂ and other dissolved gases like H₂, CO, CH₄, Ar and/or N₂. The gas may also compromise traces of methanol and other reaction byproducts from the methanol synthesis.

The term 'synthesis gas', abbreviated as 'syngas', denotes a gas comprising hydrogen, carbon dioxide, and carbon monoxide in various ratios. Optionally, the gas also comprises small amounts of other gasses, such as argon, nitrogen, and/or methane.

The term 'raw methanol' denotes a fluid containing methanol and water which has not been subject to upgrading, i.e., removal of water and/or byproducts by distillation. Optionally, the fluid also comprises small amounts of byproducts like, but not limited to, ethanol and other higher alcohols, ketones, ethers, esters, and organic acids from the synthesis of methanol. Optionally, the fluid also comprises small amounts of dissolved synthesis gas.

The term carbon 'efficiency' denotes the total number of moles carbon in the CO₂-rich feed divided by the total number of moles carbon in the methanol product.

The term hydrogen 'efficiency' denotes the total number of moles hydrogen in the hydrogen feed divided by the total number of moles hydrogen in the methanol product.

The terms 'at least a portion of' and 'at least (a) part of', in connection with a physical element, such as a feed, a stream, a section, or a unit, mean the entire element or a portion/part (fraction) thereof. Accordingly, the term 'at least partly' in connection with a physical element, such as a feed, a stream, a section, or a unit, means 'a part of' or 'the entirety of'.

The term 'section' refers normally in the specification to a subset of a plant or system.

Other definitions are provided throughout the patent application in connection with the recital of one or more embodiments of the invention.

In general terms, the methanol plant comprises:
- a CO₂-rich feed,
- a hydrogen-rich feed,
- a boiler feed water stream,
- a CO₂ electrolysis section,
- a heat exchange section,
- a methanol synthesis loop, and
- a methanol upgrading section.

Details of the components of the plant are described in the following.

The CO₂-rich feed comprises CO₂ gas, and preferably comprises at least 70% vol. CO₂, more preferably at least 90% vol. CO₂, more preferably at least 95% vol. CO₂, more preferably at least 98% vol. CO₂. The CO₂-rich feed may consist essentially of CO₂. In one embodiment, the CO₂-rich feed comprises captured CO₂, such as CO₂ captured from air, from point source emission, or from a CO₂-producing process.

The CO₂ electrolysis section is arranged to receive the CO₂-rich feed and to electrolyse at least a portion of the CO₂-rich feed to output a mixed stream comprising CO and CO₂. In one embodiment, the CO₂ electrolysis section is arranged to partially electrolyse the CO₂-rich feed to a mixture comprising CO and unconverted CO₂. In one embodiment, at least 20% of the CO2 in the CO2-rich feed is converted into CO, more preferably at least 25%, more preferably at least 30%, more preferably at least 35%, more preferably at least 40%. In one embodiment, the CO₂ electrolysis is powered with energy from a renewable energy source.

In one embodiment, the CO₂-electrolysis section comprises a solid oxide electrolysis cell (SOEC). In the electrolysis of CO₂, CO is formed at the cathode and O₂ at the anode of the electrochemical cell. The electrolysis is preferably performed at low pressure. This reduces power consumption and improves CO₂ conversion to CO. The electrolysis is preferably performed at elevated temperature. In one embodiment, the CO₂ electrolysis is carried out at 600-900°C, more preferably 700-800°C.

In one embodiment, the pressure of the CO₂-rich feed is let down before the CO₂-rich feed is received by the CO₂-electrolysis section or at the inlet of the CO₂-electrolysis section. Compressed and dried air is used in the oxy side of the electrolysis unit.

Excess heat from the mixed stream can be utilised elsewhere in the plant to reduce the dependency of the plant on the import of external heat. High temperatures at the outlet of the CO₂-electrolysis section are used to generate a low or medium pressure steam.

The heat exchange section is arranged to heat exchange at least a portion of the mixed stream with the boiler feed water stream so as to output a cooled mixed stream and a steam stream. The cooled mixed stream outputted from the heat exchange section has a lower temperature than the mixed stream received by the heat exchange section.

In one embodiment, the methanol plant comprises a purification section arranged to purify the CO₂-rich feed upstream the CO₂ electrolysis section. The CO₂-rich feed is preferably compressed before the CO₂-rich feed is purified in the purification section. The purification step serves to remove impurities, such as sulphur and/or oxygen, from the CO₂-rich feed before the feed is received by the CO₂-electrolysis section.

The methanol synthesis loop is arranged to receive at least a portion of the cooled mixed stream and the hydrogen-rich feed - preferably in admixture - and to output a raw methanol stream, a purge gas stream, and a flash gas stream. The cooled mixed stream and the hydrogen-rich feed are partly converted into methanol in one or more methanol synthesis reactors whereafter the partly converted syngas is cooled in one or more heat exchangers before it is sent to one or more gas-liquid separators. The gas-liquid separation may comprise both high-pressure and low-pressure separators.

The methanol synthesis reactors may be selected amongst adiabatic reactors, water-cooled boiling water reactors or gas-cooled reactors or combinations hereof.

In one embodiment, the purge gas stream comprises unreacted syngas. In one embodiment, the flash gas stream is a low-pressure flash gas stream. The flash gas stream is generated by degassing of the liquid methanol stream produced in the loop through pressure reduction from loop pressure to pressure in the low-pressure separator.

The hydrogen-rich feed comprises hydrogen gas, and preferably comprises at least 70% vol. H₂, more preferably at least 90% vol. H₂, more preferably at least 95% vol. H₂, more preferably at least 98% vol. H₂, more preferably at least 99% vol. H₂. The H₂-rich feed may consist essentially of H₂.

In one embodiment, the methanol synthesis loop is arranged to mix at least a portion of the cooled mixed stream and the hydrogen-rich feed, e.g. at the inlet of the methanol synthesis loop.

In one embodiment, the methanol plant comprises a compression section arranged to compress the H₂-rich feed.

In one embodiment, the CO2-rich feed and/or the hydrogen-rich feed are compressed in the methanol synthesis loop, either separately or in admixture.

In one embodiment, the hydrogen-rich feed is arranged to be obtained from electrolysis of water. The hydrogen-rich feed can be obtained from electrolysis of water using electricity optionally partly or fully from a renewable energy source. For this purpose, in one embodiment, the plant comprises an H₂O-electrolysis section and a water feedstock for the H₂O-electrolysis section. The electrolysis section is arranged to electrolyse the water feedstock to a hydrogen-rich stream, and to feed at least a portion of the hydrogen-rich stream as at least a portion of the hydrogen-rich feed to the electrical reverse water gas shift section. The hydrogen-rich feed may be produced by alkaline water electrolysis, proton exchange membrane electrolysis or anion exchange membrane electrolysis or combinations hereof.

In one embodiment, the hydrogen-rich feed is arranged to be obtained from electrolysis of water vapour (steam) in solid oxide electrolysis cell (SOEC). This reduces power consumption and improves H₂O conversion to H2. The electrolysis is preferably performed at elevated temperature. In one embodiment, the H₂O electrolysis is carried out at 600-900°C, more preferably 700-800°C.The cooled mixed stream comprises CO and CO₂. In one embodiment, the molar ratio between CO and CO₂ is the same in the mixed stream and the cooled mixed stream.

Methanol is produced in the methanol synthesis loop from syngas. In one embodiment, the methanol synthesis occurs primarily via hydrogenation of CO, as opposed to primarily via direct hydrogenation of CO₂. This is due to the higher reactivity of CO compared to CO₂.

The hydrogenation of CO can be expressed as follows:

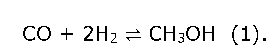

In one embodiment, a smaller part of the methanol produced in the methanol synthesis loop is formed by direct hydrogenation of CO₂. Direct hydrogen of CO₂ can be expressed as:

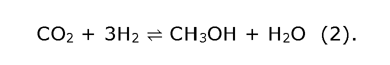

The syngas used in the methanol synthesis is produced by mixing of at least a portion of the cooled mixed stream and the hydrogen-rich feed.

The methanol upgrading section is arranged to receive at least a portion of the raw methanol stream outputted from the methanol synthesis loop and to output an upgraded methanol stream and an off-gas stream. The steam stream from the heat exchange section is arranged to provide heat energy for one or more components of the methanol upgrading section. In particular;
- at least a portion of the purge gas stream is arranged to be recycled to the mixed stream and/or the cooled mixed stream,
   and/or
- at least a portion of the flash gas stream is arranged to be recycled to at least one of: the mixed stream and/or the cooled mixed stream.

The recycling of heat energy between different sections of the plant reduces the need for import of external heat to close the heat balance of the plant.

By combining the CO₂-electrolysis section with the methanol synthesis loop and methanol upgrading section, as disclosed herein, the production of e-methanol using renewable energy, hydrogen, and captured CO₂ can be achieved. In one embodiment, the upgraded methanol stream consists essentially of grade AA e-methanol.

In one embodiment, a particular CO/CO₂ molar ratio in the mixed stream and/or the cooled mixed stream can be obtained by supplying a second CO₂-rich feed thereto.

In one embodiment, the methanol synthesis loop comprises a methanol synthesis reactor, a heat exchanger, a methanol separation section, and a compressor. The methanol synthesis loop is arranged to receive at least a portion of the cooled mixed stream and to output a raw methanol stream and a purge gas stream.

In one embodiment, the methanol synthesis loop is arranged to output a low-pressure (LP) flash gas stream. The flash gas stream is generated by degassing of the raw methanol stream produced in the loop through pressure reduction from loop pressure to pressure in low pressure separation step.

In one embodiment, the methanol synthesis loop is arranged to recycle at least a portion of the purge gas stream to the mixed stream or the cooled mixed stream, , optionally through a hydrogen recovery unit (HRU). The recycle of purge gas will increase the hydrogen efficiency of the plant. In one embodiment at least 50% of the purge gas is recycled, more preferably at least 60%, more preferably at least 70%. If the purge is recycled through a HRU essentially all the permeate stream may be fully recycled.

In one embodiment, at least a portion of the flash gas stream is recycled to the mixed gas stream and/or the cooled mixed gas stream. Suitably, at least 90% of the flash gas is recycled, more preferably at least 95%, more preferably at least 99%. The flash gas stream may essentially be fully recycled.

The methanol upgrading section is arranged to receive at least a portion of the raw methanol stream from the methanol synthesis loop and to output an upgraded methanol stream and an off-gas stream. At least a portion of the steam stream outputted from the heat exchange section is arranged to provide heat energy for one or more components of the methanol upgrading section.

In one embodiment, the methanol upgrading section comprises one or more methanol distillation columns, wherein at least a portion of the steam stream is arranged to provide heat energy for the one or more of the methanol distillation columns.

The present invention also provides a method for converting CO₂ to methanol, comprising the steps of:
a) providing a CO₂-rich feed,
b) providing a hydrogen-rich feed,
c) providing a boiler feed water stream,
d) supplying the CO₂-rich feed to a CO₂-electrolysis section,
e) electrolysing at least a portion of the CO₂-rich feed in the CO₂-electrolysis section to output a mixed stream comprising CO and CO₂ from the CO₂-electrolysis section,
f) heat exchanging at least a portion of the mixed stream with the boiler feed water stream in a heat exchange section to output a cooled mixed stream and a steam stream from the heat exchange section,
g) supplying at least a portion of the cooled mixed stream and the hydrogen-rich feed - preferably in admixture - to a methanol synthesis loop to output a raw methanol stream, a purge gas stream, and a flash gas stream, from the methanol synthesis loop,
h) supplying at least a portion of the raw methanol stream to a methanol upgrading section to output an upgraded methanol stream and an off-gas stream from the methanol upgrading section, and
i) using at least part of the steam stream to provide heat energy for one or more components of the methanol upgrading section, and
j) one or more of the following steps:
   - recycling at least a portion of the purge gas stream to the mixed stream and/or the cooled mixed stream,
      and/or
   - recycling at least a portion of the flash gas stream to the mixed stream and/or the cooled mixed stream.

In one embodiment, the CO₂-rich feed is partially electrolysed in the CO₂-electrolysis section. The mixed stream thus comprises CO formed by electrolysis of CO₂ and unconverted CO₂.

The electrolysis of CO₂ to CO is preferably performed at elevated temperature, such as between 700 and 800°C. In one embodiment, the electrolysis of CO₂ to CO is operated at a pressure between 0.3-5 bar g, more preferably 0.5-3 bar g. This reduces power consumption and improves CO₂ conversion to CO.

In one embodiment, the method further comprises the steps of:
carrying out electrolysis of a water feedstock in an H₂O-electrolysis section to generate a hydrogen-rich stream,
feeding at least a portion of the hydrogen-rich stream as at least a portion of the hydrogen-rich feed to the methanol synthesis loop.

In one embodiment, at least a portion of the CO₂ in the CO₂-rich feed is captured CO₂ from a point source or extracted from the air.

In one embodiment, the electrolysis in the CO₂-electrolysis section of at least a portion of the CO₂-rich feed is powered with energy from a renewable energy source.

In one embodiment, all processes, including CO₂ electrolysis, methanol synthesis, and raw methanol upgrade, are powered with energy from a renewable energy source.

In one embodiment, the admixture received by the methanol synthesis loop has a molar module $M = \frac{{H}_{2} - {CO}_{2}}{CO + {CO}_{2}}$ of between 1.5 and 2.5, preferably around 2.0, more preferably 2.01.

The method comprises mixing at least a portion of the cooled mixed stream and the hydrogen-rich feed to produce a syngas stream and using at least a portion of the syngas stream in the methanol synthesis loop for production of the raw methanol stream. In one embodiment, the mixing takes place upstream the methanol synthesis loop and upstream the purification section. In another embodiment, the mixing takes place upstream the methanol synthesis loop and downstream the purification section. After the mixing step, at least a portion of the syngas stream is supplied to the methanol synthesis loop to output a raw methanol stream and a purge gas stream. In another embodiment, the mixing step takes place in the methanol synthesis loop or at the inlet thereof.

In one embodiment, the raw methanol stream is produced from a syngas stream produced by mixing at least a portion of the cooled mixed stream and at least a portion of the hydrogen-rich feed, the syngas stream having a molar module $M = \frac{{H}_{2} - {CO}_{2}}{CO + {CO}_{2}}$ of between 1.5 and 2.5, preferably around 2.0, more preferably 2.01.

In one embodiment, the upgraded methanol stream consists essentially of methanol produced from renewable energy.

In one embodiment, the method uses the methanol plant as disclosed herein.

Several benefits can be achieved with the embodiments disclosed herein, compared to methanol production by direct hydrogenation of CO₂. Some of the benefits are e.g.:
- reduction in hydrogen feed per kg of methanol product;
- reduction in CO₂ feed per kg of methanol product;
- reduction in steam requirement within the plant and hence reduced power requirement;
- less catalyst in the methanol synthesis, smaller methanol synthesis loop, and smaller methanol synthesis reactor. This is due to the higher reactivity of CO compared to CO₂.

The plant provides a low-emission and cost-effective solution to methanol production and allows for substantially complete CO₂ utilisation. A CO₂-to-methanol carbon efficiency above 95%, such as above 98%, can be achieved. Grade AA methanol can thus be produced using electricity from renewable energy, electrolytic H₂, and captured CO₂.

### Specific embodiments

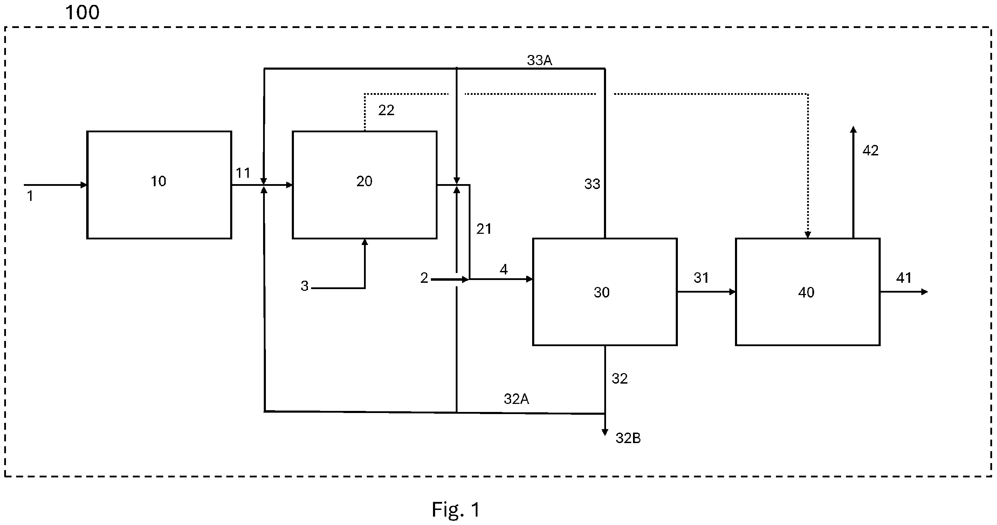

Fig. 1 shows a first embodiment of the methanol plant (100) disclosed herein, the methanol plant comprising:
- a CO₂-rich feed (1),
- a hydrogen-rich feed (2),
- a boiler feed water stream (3),
- a CO₂-electrolysis section (10),
- a methanol synthesis loop (30), and
- a methanol upgrading section (40).

The CO₂-electrolysis section (10) arranged to receive the CO₂-rich feed (1) and to partially electrolyse it, to output a mixed stream (11) comprising CO and unconverted CO₂. The heat exchange section (20) is arranged to heat exchange at least a portion of the mixed stream (11) with the boiler feed water stream (3) so as to output a cooled mixed stream (21) and a steam stream (22). The methanol synthesis loop (30) which comprises one or more methanol synthesis reactors, e.g., a boiling water reactor (BWR), adiabatic reactor, gas cooled reactor or combinations hereof, is arranged to receive at least a portion of the cooled mixed stream (21) and the hydrogen-rich feed (2) - preferably in admixture (4) - and to output a raw methanol stream (31), a purge gas stream (32), and a flash gas stream (33).

In the illustrated embodiment, at least a portion of the cooled mixed stream (21) and the hydrogen-rich feed (2) are mixed to form an admixture (4) which is sent to the methanol synthesis loop (30). The admixture preferably has a molar module $M = \frac{{H}_{2} - {CO}_{2}}{CO + {CO}_{2}}$ of between 1.5 and 2.5, preferably around 2.0, more preferably 2.01. In an alternative, the portion of the cooled mixed stream (21) and the hydrogen-rich feed (2) are mixed at the inlet of the methanol synthesis loop (30) or in the methanol synthesis loop (30).

At least a portion (32A) of the purge gas stream (32) from the methanol synthesis loop (30) is arranged to be recycled to the mixed stream (11) and/or the cooled mixed stream (21). Additionally or alternatively, at least a portion (33A) of the flash gas stream (33) is arranged to be recycled to the mixed stream (11) and/or the cooled mixed stream (21).

The methanol upgrading section (40) is arranged to receive at least a portion of the raw methanol stream (31) and to output an upgraded methanol stream (41) and an off-gas stream (42), wherein at least a portion of the steam stream (22) is arranged to provide heat energy for one or more components of the methanol upgrading section (40).

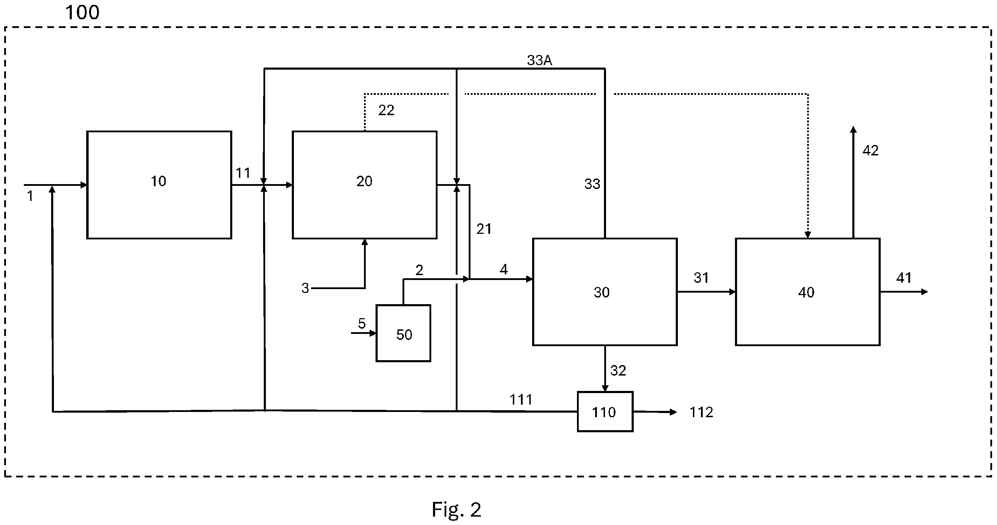

The layout illustrated in Figure 2 additionally includes an H₂O-electrolysis section (50) and a water feedstock (5) to the H₂O-electrolysis section (50). The H₂O-electrolysis section (50) is arranged to electrolyse the water feedstock (5) to a hydrogen-rich stream (51), and to feed at least a portion of the hydrogen-rich stream (51) as at least a portion of the hydrogen-rich feed (2) to the methanol synthesis loop (30).

The layout illustrated in Figure 2 additionally includes a hydrogen recovery unit (HRU, 110) arranged to receive at least part of the purge gas stream (32) and separate it into a hydrogen rich stream (111) and a hydrogen depleted stream (112). If an HRU is installed, the hydrogen rich stream (111) is returned to the cooled mixed stream (21) (preferred), the mixed stream (11) and/or the CO₂-rich feed (1).

### Examples

**Table 1 shows a comparison of methanol production via direct hydrogenation of CO₂ and methanol production according to the plant and method disclosed herein, based on calculated heat and mass balances. Energy from steam per ton of methanol product has been calculated based on the steam flow and its saturation enthalpy.**

| | **Methanol production via hydrogenation of CO₂** | **Methanol production according to the plant and method disclosed herein** |
|---|---|---|
| Gas Reactivity (CO/CO₂) at BWR Inlet | 0.2 | 0.6 |
| Energy consumption for distillation, MWh/t methanol | 1.17 | 1.15 |
| Available steam (energy) from synthesis, MWh/t methanol | 0.22 | 0.51 |
| Imported saturated LP Steam (energy) @ 8.0 bar g, MWh/ton methanol | 0.95 | 0.64 |

**Table 2 shows a comparison of methanol production according to the plant and method disclosed herein with and without the recycle of flash gas, based on calculated heat and mass balance for the plant and method shown in Fig. 2.**

| | **Methanol production according to the plant and method disclosed herein without recycle** | **Methanol production according to the plant and method disclosed herein with recycle** |
|---|---|---|
| Flash gas recycle, Nm3/h | 0 | 155 |
| Purge gas recycle, Nm3/h | 0 | 0 |
| Specific H2 feed consumption, Nm3/MT MeOH | 1865 | 1863 |
| Specific CO2 feed consumption, Nm3/MT MeOH | 719 | 715 |

## Claims

1. A methanol plant (100), the methanol plant (100) comprising:
- a CO₂-rich feed (1),
- a hydrogen-rich feed (2),
- a boiler feed water stream (3),
- a CO₂-electrolysis section (10) arranged to receive the CO₂-rich feed (1) and to electrolyse at least a portion of the CO₂-rich feed to output a mixed stream (11) comprising CO and CO₂,
- a heat exchange section (20) arranged to heat exchange at least a portion of the first mixed stream (11) with the boiler feed water stream (3) so as to output a cooled mixed stream (21) and a steam stream (22),
- a methanol synthesis loop (30) arranged to receive at least a portion of the cooled mixed stream (21) and the hydrogen-rich feed (2) - preferably in admixture (4) - and to output a raw methanol stream (31), a purge gas stream (32), and a flash gas stream (33),
- a methanol upgrading section (40) arranged to receive at least a portion of the raw methanol stream (31) and to output an upgraded methanol stream (41) and an off-gas stream (42),
wherein at least a portion of the steam stream (22) is arranged to provide heat energy for one or more components of the methanol upgrading section (40) and
wherein
at least a portion (32A) of the purge gas stream (32) is arranged to be recycled to the mixed stream (11) or the cooled mixed stream (21),
and/or
at least a portion (33A) of the flash gas stream (33) is arranged to be recycled to the mixed stream (11) or the cooled mixed stream (21).

2. The methanol plant (100) according to claim 1, further comprising an H₂O-electrolysis section (50) and a water feedstock (5) to the H₂O-electrolysis section (50), the H₂O-electrolysis section (50) being arranged to electrolyse the water feedstock (5) to a hydrogen-rich stream (51), and to feed at least a portion of the hydrogen-rich stream (51) as at least a portion of the hydrogen-rich feed (2) to the methanol synthesis loop (30).

3. The methanol plant (100) according to claim 1 or 2, wherein the flash gas stream (33) is a low-pressure flash gas stream.

4. The methanol plant (100) according to any one of the preceding claims, wherein the purge gas stream (32) comprises unreacted syngas.

5. The methanol plant (100) according to any one of the preceding claims, wherein the methanol upgrading section (40) comprises one or more methanol distillation columns, wherein at least a portion of the steam stream (22) is arranged to provide heat energy for one or more of the methanol distillation columns.

6. The methanol plant (100) according to any one of the preceding claims, wherein the plant additionally includes a hydrogen recovery unit (110) arranged to receive at least a portion the purge gas stream (32) and separate it into a hydrogen rich stream (111) and a hydrogen depleted stream (112), preferably wherein the hydrogen rich stream (111) is recycled to the mixed stream (11), the cooled mixed stream (21) and/or the CO₂-rich feed (1).

7. The methanol plant (100) according to any one of the preceding claims, wherein at least a portion of the steam stream (22) is arranged to be provided as steam rich feed for production of a least a part of the hydrogen-rich feed (2) in a high temperature electrolyser.

8. A method for converting CO₂ to methanol, comprising the steps of:
a) providing a CO₂-rich feed (1),
b) providing a hydrogen-rich feed (2),
c) providing a boiler feed water stream (3),
d) supplying the CO₂-rich feed (1) to a CO₂-electrolysis section (10),
e) electrolysing at least a portion of the CO₂-rich feed (1) in the CO₂-electrolysis section (10) to output a mixed stream (11) comprising CO and CO₂ from the CO₂-electrolysis section (10),
f) heat exchanging at least a portion of the first mixed stream (11) with the boiler feed water stream (3) in a heat exchange section (20) to output a cooled mixed stream (21) and a steam stream (22) from the heat exchange section (20),
g) supplying at least a portion of the cooled mixed stream (21) and the hydrogen-rich feed (2) - preferably in admixture (4) - to a methanol synthesis loop (30) to output a raw methanol stream (31), a purge gas stream (32), and a flash gas stream (33), from the methanol synthesis loop (30),
h) supplying at least a portion of the raw methanol stream (31) to a methanol upgrading section (40) to output an upgraded methanol stream (41) and an off-gas stream (42) from the methanol upgrading section (40), and
i) using at least a portion of the steam stream (22) to provide heat energy for one or more components of the methanol upgrading section (40),
and
j) one or more of the following steps:
- recycling at least a portion of the purge gas stream (32) to the mixed stream (11) and/or the cooled mixed stream (21),
and/or
- recycling at least a portion of the flash gas stream (33) to the methanol mixed stream (11) or the cooled mixed stream (21).

9. The method according to claim 8, further comprising the steps of:
carrying out electrolysis of a water feedstock (5) in an H₂O-electrolysis section (50) to generate a hydrogen-rich stream (51), and
feeding at least a portion of the hydrogen-rich stream (51) as at least a portion of the hydrogen-rich feed (2) to the methanol synthesis loop (30).

10. The method according to any one of claims 8-9, wherein the admixture (4) has a molar module $M = \frac{{H}_{2} - {CO}_{2}}{CO + {CO}_{2}}$ of between 1.5 and 2.5, preferably around 2.0, more preferably 2.01.

11. The method according to any one of claims 8-10, the method comprising mixing at least a portion of the cooled mixed stream (21) and the hydrogen-rich feed (2) to produce a syngas stream, and using at least a portion of the syngas stream in the methanol synthesis loop to produce the raw methanol stream (31).

12. The method according to any one of claims 8-11, wherein the electrolysis in the CO₂-electrolysis section (10) of at least a portion of the CO₂-rich feed (1) is powered with energy from a renewable energy source.

13. The method according to any one of claims 8-12, wherein the plant additionally includes a hydrogen recovery unit (110), said method further comprising the steps of:
- separating at least a portion of the purge gas stream (32) in said hydrogen recovery unit (110) into a hydrogen rich stream (111) and a hydrogen depleted stream (112), and
- recycling the hydrogen rich stream (111) to the mixed stream (11), the cooled mixed stream (21) and/or the CO₂-rich feed (1).

14. The method according to any one of claims 8-13, wherein at least a portion of the steam stream (22) is provided as steam rich feed for production of a least a part of the hydrogen-rich feed (2) in a high temperature electrolyser.
